# NEUE EUROPÄISCHE PATENTSCHRIFT

(11) **EP 0 810 887 B2**
(45) Veröffentlichungstag und Bekanntmachung des Hinweises auf die Entscheidung über den Einspruch: **17.10.2007**
(45) Hinweis auf die Patenterteilung: 28.08.2002
(21) Anmeldenummer: 96904770.3
(22) Anmeldetag: 14.02.1996
(51) Int. Cl.: A61L 15/60, C08J 5/18, B32B 5/18

(54) **FLÄCHENFÖRMIGE, SUPERABSORBIERENDE GEBILDE**
SHEET-LIKE SUPERABSORBENT STRUCTURES
STRUCTURES SUPER-ABSORBANTES EN FORME DE FEUILLES

(30) Priorität: 20.02.1995 DE 19505708
(43) Veröffentlichungstag der Anmeldung: 10.12.1997
(73) Patentinhaber: Stockhausen GmbH, 47805 Krefeld (DE)
(72) Erfinder: BRÜGGEMANN, Helmut, D-47057 Duisburg (DE); DAHMEN, Kurt, D-41239 Mönchengladbach (DE); LEHWALD, Dieter, D-50931 Köln (DE); THEILMANN, Roland, D-47805 Krefeld (DE)
(74) Vertreter: Kahlhöfer, Hermann
(86) Internationale Anmeldenummer: PCT/EP1996/000621
(87) Internationale Veröffentlichungsnummer: WO 1996/025959

(56) Entgegenhaltungen:
- EP-A- 0 378 940
- EP-A- 0 624 618
- WO-A-94/25519
- JP-A- 182 362
- JP-A- 622 918
- JP-A- 5 964 650
- JP-A- 6 315 501
- JP-A- 57 208 236
- JP-A- 63 227 645
- US-A- 4 335 722
- US-A- 4 413 995
- US-A- 5 382 610
- US-P- 4 066 583
- DATABASE WPI Section Ch, Week 7710 Derwent Publications Ltd., London, GB; Class A89, AN 77-17578Y XP002010294 & JP,A,52 012 395 (SANYO CHEM IND KK) , 29.Januar 1977 in der Anmeldung erwähnt

## Beschreibung

Die Erfindung betrifft flächenförmige Absorptionsmittel für Wasser und wäßrige Lösungen, Verfahren zur Herstellung der flächenförmigen Absorptionsmittel sowie deren Verwendung.

Flächenförmige Absorptionsmittel eröffnen die Möglichkeit, superabsorbierende Polymere (SAP) in Pulver- oder Granulatform in beliebiger Anordnung in/auf eine bzw. mehrere Flächen zu fixieren. Diese Fixierung geschieht dann in optimaler Form für den späteren Anwendungszweck wie für Babywindeln, Erwachseneninkontinenz, für Kabelummantelungen, für Bodenverbesserer, für Verpackungsbeilagen in der Nahrungsmittelindustrie, für die Tierhygiene, für Wundabdeckungen und für Wischtücher.

Um eine für den späteren Anwendungszweck optimale Verteilung eines pulverförmigen, superabsorbierenden Polymeren zu erreichen und diese zu fixieren, wurden verschiedene Möglichkeiten beschrieben.

In EP 212 618 B1 werden Windelkonstruktionen beschrieben, in denen Polymerisate mit bestimmter Korngrößenverteilung in einer Cellulosefaserschicht verteilt werden. Eine solche Konstruktion ist jedoch bezüglich der Verteilung des superabsorbierenden Polymeren nicht hinreichend stabil, insbesondere kann sich die Verteilung des SAPs während des Transportes in unerwünschter Weise ändern, wodurch beispielsweise in einer Windel eine ungleichmäßige Absorption erfolgt.

Eine weitere Möglichkeit pulverförmige, superabsorbierende Polymeren in einer bestimmten Anordnung zu fixieren, beschreibt EP 425 269 A2, derzufolge die SAPs an thermoplastische, wasserunlösliche Fasern angebunden werden. Das Anbinden des SAPs an die Faser geschieht dann in der Form, daß eine oberflächlich angeschmolzene Faser mit dem pulverförmigen, superabsorbierenden Polymeren in Kontakt gebracht wird. Die Fasern ihrerseits werden untereinander in gleicher Form fixiert. Der Nachteil dieses Verfahrens besteht darin, daß die Absorptionskapazität des pulverförmigen, superabsorbierenden Polymeren nicht voll ausgenutzt wird. Ein Teil des SAPs wird vom thermoplastischen Kunststoff überzogen und dann von Wasser oder wäßrigen Lösungen nicht mehr erreicht.

EP 547 474 A1 beschreibt ein Verfahren zur Herstellung von absorbierenden Materialien, in denen superabsorbierende Polymere verteilt sind. Die so erhaltenen absorbierenden Materialien besitzen eine Absorptionskapazität, die geringer ist, als es dem Anteil des in den Materialien eingearbeiteten SAP entsprecht, d.h. aufgrund der Auswahl der verwendeten Materialien und des angewendeten Herstellungsprozesses ein Teil des SAP blockiert. Die Art des verwendeten Matrixmaterial ist auch insofern eingeschränkt, daß der Schmelzpunkt dieses Materials über der Zersetzungstemperatur des SAP liegen muß.

Die EP 303 445 A1 beschreibt ein absorbierendes Flächengebilde, in dem auf einem Träger ein wasserhaltiges SAP fixiert wurde. Die Verwendung diese Gebildes ist auf ein Pflaster als Medikamentenreservoir eingeschränkt.

Die JP Appl No. 75 - 85462 beschreibt ein Verfahren zur Herstellung von superabsorbierenden Flächen aus einem Stärke - Pfropfpolymeren, das in einem wasserlöslichen, filmbildenden Polymeren eingebunden ist.
Als unverzichtbarer dritter Bestandteil wird in dieser Druckschrift ein Material genannt, das als Basismaterial fungiert. Das superabsorbiernde Polymere wird mit dem löslichen, filmbildenden Polymeren auf diesem Basismaterial fixiert.

EP 604 730 A1 beschreibt SAP enthaltende Gebilde, die in Wasser zerfallen. Neben dem SAP sind dispergierbare Polymere und Weichmacher als unverzichtbare Komponeten genannt. Keinesfalls erfüllen die gemäß dieser Anmeldung hergestellten Gebilde die Forderung nach einer definierten Anordnung eines Superabsorbers in einer Matrix, da die in dieser Schrift beschriebenen Verfahren wie Extrudieren, Mischen oderVermengen dazu völlig ungeeignet sind.
Nach dem Desintegrieren der beschriebenen Flächengebilde verbleiben neben dem Superabsorber auch Partikel, das Matrixmaterial ist also nicht wasserlöslich.

Aufgabe der vorliegenden Erfindung ist es daher, eine superabsorbiernde Fläche mit einer definierten Anordung superabsorbierender Polymere bei voller Ausschöpfung des Quellvermögens der superabsorbierenden Polymeren bereitzustellen, d. h. ohne kapazitative Einbußen des Absorptionsvermögens.

Eine weitere Aufgabe der Erfindung ist es, Herstellungsverfahren zu finden, nach denen eine solche Fläche auf einfachem und kostengünstigem Wege herzustellen ist. Außerdem sollte die Fläche nicht, wie in der japanischen Patentanmeldung Nr. 75 - 85462 beschrieben, auf einem Untergrund fixiert sein, so daß die Verwendung einer solchen Fläche universeller bleibt.

Die Aufgabe wird gelöst durch ein flächenformiges Absorptionsgebilde für Wasser oder wäßrige Flüssigkeiten, enthaltend
A) wenigstens ein wasserquellbares, synthetisches und/oder natürliches Polymer und
B) wenigstens ein wasserlösliches, synthetisches und/oder natürliches Polymer,
wobei die Komponente A in der flächenförmig ausgebildeten Matrixkomponente B definiert ein- oder angebunden ist und wobei das flächenförmige Absorptionsmittel erhältlich ist durch ein Verfahren, bei dem man aus dem wenigstens einen wasserlöslichen synthetischen und/oder natürlichen Polymeren (B) eine flächenförmige Matrix ausbildet und diese mit dem wasserquellbaren synthetischen und/oder natürlichen Polymeren (A) versieht.

Durch diese Kombination wird die Aufnahmekapazität des superabsorbierenden Polymers nicht reduziert, da sich die Matrix beim Kontakt mit Wasser oder wässrigen Flüssigkeiten löst und somit das Quellen des SAP nicht behindert. Durch diese Ausführung ist es möglich, die Aufnahmegeschwindigkeit des erfindungsgemäßen Absorptionsmittels für Wasser oder wäßrige Flüssigkeiten zu steuern, außerdem kann die Flexibilität eines solchen Absorptionskörpers je nach beabsichtigtem Einsatz eingestellt werden.

Als Grundlage für die Matrix kommen sowohl synthetische, wasserlösliche, filmbildende Polymere wie Polyvinylalkohole, Polyalkylallylether, Polyglykolether, Polyvinylpyrolidone, Polyacrylate , Polymethacrylate sowie deren Derivate und Copolymere sowie natürliche, wasserlösliche, filmbildende Polymeren wie Guar, Alginate, Agar Agar, Xanthan, Pektin und Stärke u.ä. sowie chemisch modifizierte Rohstoffe wie Ether und/oder Ester und/oder Hydrolysate und/oder Oxidationsprodukte von Polysacchariden oder Proteinen wie Cellulose, Amylose, Stärke oder Weizenkleber in Frage sowie Copolymerisate und/oder Pfropfpolymerisate auf Basis natürlicher oder synthetischer Polymere.
Die Auswahl des Matrixmaterials hängt nicht zuletzt vom beabsichtigten Einsatzzweck ab. Durch das Matrixmaterial läßt sich die Flexibilität der superabsorbierenden Fläche in weiten Bereiche variieren. Bei vorgegebener Matrix kann die Flexibilität der superabsorbierenden Fläche auch durch Zuschlagstoffe wie Weichmacher oder plastifizierende Mittel wie 2-Ethylhexanol, Glycerin und Phthalsäureester, jedoch auch durch Füllstoffe wie Kreide, Pigmente und Fasern verändert werden.

Die Basis des eingesetzten SAPs kann sowohl ein synthetischer Stoff wie ein wasserquellbares Polymer und/oder Copolymer auf Basis von (Meth)-acrylsäure, (Meth-)acrylnitril, (Meth-)acrylamid, Vinylacetat, Vinylpyrrolidon, Vinylpyridin, Maleinsäure(anhydrid), Itakonsäure (-anhydrid), Fumarsäure, Vinylsulfonsäure sowie die Salze, die Amide, die N-Alkylderivate, die N,N-dialkylderivate und die Ester der polymerisierbaren 5Säuren, als auch ein Stoff nativen Ursprungs wie Produkte aus Guarkernmehl, Carboxymethylcellulose, Xanthan, Alginate, Gummi Arabicum, Hydroxyethylcellulose, Hydroxypropylcellulose, Methylcellulose, Stärke und Stärkederivate sowie teilweise vernetzte Produkte daraus sein. Es können auch Mischungen oder Copolymerisate bzw. Propfpolymerisate der zuvor genannten Komponenten eingesetzt werden.
Bevorzugte Materialien sind teilneutralisierte, schwachvernetzte Polymere und Copolymere der Acrylsäure und des Acrylamids, Pfropfpolymerisate der Stärke, sowie vernetzte Stärken und Cellulosederivate. Geeignete Produkte sind z.B. die kommerziell erhältlichen FAVOR®- und Stockosorb®-Typen der Chemischen Fabrik Stockhausen GmbH.

Die Komverteilung des eingesetzten pulverförmigen, superabsorbierenden Polymeren kann in weiten Grenzen variieren - es kommen Körner im Bereich von 0,1 µm bis zu 20.000 µm in Betracht. Bevorzugt sind solche Kornfraktionen, die in einem Bereich von 1µm bis zu 5000µm liegen. Besonders bevorzugt sind solche Kornfraktionen, die in einem Bereich von 20 bis 1000 µm liegen.
Die Kornfraktion an SAP, die in die Fläche eingebracht, wird richtet sich wesentlich nach dem für die Folie vorgesehenen Anwendungszweck. Während in Windeln für gewöhnlich Kornfraktionen von etwa 500µm verwendet werden, werden in der Landwirtschaft Kornfraktionen um 1000µm, in der Kabelindustrie Korngrößen um 100µm und geringer bevorzugt.

Die Herstellung der superabsorbierenden Fläche geschieht erfindungsgemäß durch die Kombination der zuvor genannten Komponenten. Dazu wird durch geeignete Verfahren wie Sprühen, Streichen und Rakeln eine viskose Lösung des wasserlöslichen Polymeren auf eine Fläche wie ein Metallblech, silikonisiertes Papier oder eine PTFE - Folie aufgebracht.

Diese Fläche kann sowohl ein ebenes, großflächiges Gebilde als auch die Oberfläche einer Kugel sein.

Anschließend wird das wasserlösliche Polymer auf der Fläche mit einem superabsorbierenden Polymeren bestreut. Das so erhaltene Produkt wird bei geeigneten Temperaturen, d. h. bei Temperaturen zwischen den üblicherweise beim Gefriertrocknen angewandten Temperaturen und 300°C, vorzugsweise bei Temperaturen zwischen 50°C und 240°C, gegebenenfalls unter vermindertem Druck, getrocknet.
Zum Trocknen des Flächengebildes können auch die Mikrowellentechnik oder Gefriertrocknungstechniken angewendet werden.

Im Zuge der Herstellung der absorbierenden Flächengebilde, insbesondere bei deren Trocknung, kann es gegebenenfalls zu chemischen bzw. physikalischen Bindungen zwischen dem Matrixmaterial B und der absorbierenden Komponente A kommen. Als Beispiel für eine chemische Bindung sei hier die Veresterungsreaktion genannt, die zwischen Carboxyl- und Hydroxylgruppen stattfinden kann. Physikalische Bindungen ergeben sich z.B. durch Verschlaufungen oder Verhakungen der Polymermoleküle im Oberflächenbereich der Komponente A bzw. durch Wechselwirkungen funktioneller Gruppen der Polymermoleküle in den Komponenten A und B.

Nach dem Trocknen wird das superabsorbierende Flächengebilde von der verwendeten Hilfsoberfläche abgelöst.

Eine weitere bevorzugte Herstellung der superabsorbierenden Fläche geschieht erfmdungsgemäß, indem eine Folie oder eine andere, aus dem Matrixmaterial gebildete Fläche mit einem SAP bestreut wird und die Mischung anschließend mit Wasser oder einer wässrigen Lösung oder einem anderen Lösungsmittelgemisch befeuchtet wird, oder indem eine Folie oder eine andere, aus dem Matrixmaterial gebildete Fläche mit einem befeuchteten SAP bestreut wird und diese Fläche dann anschließend getrocknet wird.

Eine weitere bevorzugte Herstellung der superabsorbierenden Fläche geschieht erfindungsgemäß, indem eine Folie oder eine andere, aus dem Matrixmaterial gebildete Fläche mit einem SAP bestreut wird und diese Mischung bis zur Erweichung des Matrixmaterials erhitzt wird. Die so gebildete Fläche kann dann noch, um das superabsorbierende Polymer besser in der Matrix zu fixieren, kalandriert werden. Voraussetzung für dieses Verfahren ist, daß das verwendete Matrixmaterial Thermoplastizität besitzt.

Eine weitere bevorzugte Herstellung eines erfindungsgemäßen absorbierenden Flächengebildes geschieht durch Extrudieren des Matrixmaterials. Das SAP wird nach dem Extrudieren dem Matrixmaterial in geeigneter Form (bezüglich der Korngröße und des Dosiervorgangs) zugeführt.

Eine solche definierte Anordnung ist während des Herstellungsvorgangs leicht durch das Benutzen einer Schablone bzw. durch bestimmte Streuanordnungen während des Aufbringens des pulverförmigen, superabsorbierenden Polymeren zu realisieren. Durch Schichtung mehrerer superabsorbierender Flächen lassen sich räumlich definierte Absorberstrukturen schaffen, die zudem einen Absorptionsgradienten aufweisen können.

Optional kann ein erfindungsgemäßes Flächengebilde auch direkt während der Herstellung einer wasserlöslichen Folie gebildet werden, d.h. daß der Superabsorber bei einem geeigneten Verfahrensschritt während der Herstellung der flächenförmigen Matrix direkt in diese eingebracht wird.

Das Verhältnis von superabsorbierendem Polymer und Matrix kann in weiten Grenzen variiert werden, es kann zwischen Matrix : SAP = 1 : 1000 bis 100 : 1 liegen. Bevorzugt sind Verhältnisse von Matrix : SAP = 1 : 100 bis 10 : 1, besonders bevorzugt sind Verhältnisse von Matrix : SAP = 1 : 25 bis 2 : 1.
Während bei der Konstruktion von Windeln in der Regel hohe SAP - und geringe Matrixkonzentrationen wünschenswert sind, werden in anderen Bereichen für Pflanzgefäße oder für Beilagen in Lebensmittelverpackungen oft geringere SAP - Konzentrationen bevorzugt.
Das Verhältnis von SAP/Matrix richtet sich also im wesentlichen nach dem beabsichtigten Einsatzzweck.

Die Dicke der absorbierenden Fläche läßt sich sowohl über die Menge der eingesetzten Matrix beeinflussen als auch über die Körnung und die Menge an eingesetztem SAP. Sie kann zwischen 0,2 µm und 30.000 µm liegen, bevorzugt sind solche Flächen, die eine Dicke von 1 bis 6000 µm aufweisen, besonders bevorzugt sind Flächendicken zwischen 20 µm und 2000µm. Auch die Dicke der absorbierenden Fläche muß je nach beabsichtigten Einsatzweck angepaßt werden. So muß die Schichtdicke einer Fläche, welche für Windelkonstruktionen oder in der Damenhygiene vorgesehen ist , möglichst gering sein, um den Tragekompfort der Windel zu erhöhen. Bei Flächen, die für eine Depotzubereitung vergesehen sind, kann eine höhere Dicke zu einer wünscheswerten Verzögerung der Freisetzung des Depotmaterials führen.

Überraschenderweise wurde nun festgestellt, daß solche Flächen ein Absorptionsvermögen fiir Wasser oder wäßrige Lösungen besitzen, wie es dem Anteil an eingebrachtem superabsorbierenden Material entspricht. Ein kapazitativer Verlust im Aufnahmevermögen des Superabsorbers, wie er aufgrund der Anwesenheit des Matrixmaterials zu erwarten ist, entsteht nicht. Dies gilt sowohl für die Gesamtaufnahme als auch fiir die Retention (Aufnahme mit anschließender Druckbelastung) als auch für Aufnahmen während einer Druckbelastung (Absorption under Load).

Weiterhin wurde überraschend festgestellt, daß die Aufnahmegeschwindigkeit für Wasser oder wässrige Lösungen solcher Flächen vom Verhältnis des Matrixmaterials zum superabsorbierenden Polymeren abhängt. Die Aufnahmegeschwindigkeit läßt sich also durch das Verhältnis SAP/Matrix und natürlich auch durch die Art des Matrixmaterials steuern und zwar in der Weise, daß ein höherer Matrixanteil zu einer Reduktion der Aufnahmegeschwindigkeit führt.

Die erfindungsgemäßen, superabsorbierenden Flächen erfüllen die Forderung nach einer definierten Anordnung des superabsorbierenden Polymeren auf einer Fläche, wie sie vorteilhaft in Hygieneartikeln anwendbar ist. Die Vorteile der definierten Anordnung von Superabsorbem in Hygieneartikeln sind in der EP 212 618 B1 beschrieben und ergeben sich beispielsweise daraus, daß die Flüssigkeitsbelastung in einer Windel uneinheitlich ist. Somit sind in einer Windel Stellen mit hoher und niedriger SAP Konzentration erforderlich.

### Testmethoden:

### Teebeuteltest (TBT)

Zur Bestimmung des Absorptionsvermögens wurde der TBT durchgeführt. Als Prüflösung wurde (soweit nicht anders erwähnt) eine 0,9%ige NaCl-Lösung verwendet.

Aus der absorbierenden Fläche wird ein Stück Material ausgestanzt, das etwa 0,2 g des SAPs enthält. Dieses Stück wird in einem Teebeutel eingewogen. Anschließend wird der Teebeutel für eine definierte Zeit in die Testlösung gelegt. Nach fünfminütiger Abtropfzeit wurde der Teebeutel ausgewogen (Bestimmung des TBT max.), anschließend wurde der Teebeutel in einer Zentrifuge (handelsübliche Wäscheschleuder, 1400 Upm) abgeschleudert. Danach wurde wiederum ausgewogen (Bestimmung des TBT ret.).
Durch mehrere Tests mit dem selben Material und unterschiedlichen Tauchzeiten kann die Aufnahme als Funktion der Tauchzeit (Aufnahmegeschwindigkeit) des superabsorbiemeden Flächengebildes für Wasser bzw. wässrige Lösungen bestimmt werden.
Die Flüssigkeitsaufnahme wird entweder auf 1 g der Fläche, auf 1 g des eingesetzten SAPs oder auf 1 m² der Fläche berechnet.

### Absorption under Load (AUL)

Um das Flüssigkeitsaufnahmevermögen unter Druck zu bestimmen, wurde die "Absorption under Load", wie in der EP 339 461 beschrieben, bestimmt.

Abweichend von dieser Vorschrift wurde ein kreisrundes Stück des superabsorbierenden Körpers von der Größe des Innendurchmessers des AUL-Tiegels als Prüfsubstanz eingesetzt. Die Flüssigkeitsaufnahme wurde entweder auf 1 g des Körpers, auf 1 g des eingesetzten SAPs oder auf 1 m² des Körpers berechnet.

### Figurenbeschreibung:

### Figur 1

Streurahmen
1 (helles Feld) durchlässiger Teil des Streurahmens
2 (dunkles Feld) undurchlässiger Teil des Streurahmens

### Figur 2

Windelkonstruktion
1 Laminate aus Poly(propylen) - Abdeckvlies und Poly(ethylen) - Folie
2 Auslaufschutz mit eingearbeiteten Gummifäden
3 Abdeckvlies aus Poly(propylen)
4 Poly(ethylen) - Folie auf der Rückseite
5 Umhüllung des Kerns aus Cellulosefasern
6 Kern, der das superabsorbierende Flächengebilde enthält

Die Erfindung wird an den nachstehenden Beispielen erläutert.

### Beispiele 1- 3

Aus 25 g Vinol®205 (wasserlöslicher Polyvinylalkohol) und 75 g deionisiertem Wasser wird eine hochviskose Lösung hergestellt. Ein Teil dieser Lösung (vgl. Tabelle) wird auf einer Fläche (teflonisierte Folie o.ä.) von 270 cm² gleichmäßig ausgestrichen. Die so entstandene Fläche wird mit ca. 30 g Superabsorber FAVOR®SXM 100 (schwachvernetztes, teilneutralisiertes Polyacrylat) bestreut und anschließend für 5 Minuten bei einer Temperatur von 180°C getrocknet. Dann wird mit einem Pinsel der nicht fixierte Teil des Superabsorbers entfernt. Es werden flexible, superabsorbierende Flächen erhalten, die sich leicht von der Oberfläche (teflonisierte Folie) ablösen lassen.

**Tabelle 1:**

| Die Tabelle zeigt die Abhängigkeit der Aufnahmegeschwindigkeit vom Verhältnis Matrix : SAP, wobei mit sinkendem Matrixanteil die Aufnahme schneller wird. | | | | | | |
|---|---|---|---|---|---|---|
| Bsp. | Lösung | SAP | Vinol®205 | TBT (30 sek.) | TBT (60 sek. ) | TBT (30 min.)∗ |
| | | | | max./ret. | max./ret. | max./ret. |
| | [g] | [g/m²] | [g/m²] | [g/g]/[g/g] | [g/g]/[g/g] | [g/g]/[g/g] |
| 1 | 10 | 226 | 93 | 6/6 | 9/9 | 50/31 |
| 2 | 5 | 182 | 46 | 7/7 | 12/12 | 50/31 |
| 3 | 2.5 | 203 | 23 | 9/9 | 13/13 | 50/31 |
| SXM 100 | 0 | ------ | 0 | 9/9 | 14/14 | 50/31 |
| (die mit ∗ gekennzeichneten TBT Werte beziehen sich auf die Menge an eingesetztem Superabsorber, die anderen TBT Werte beziehen sich auf das Flächengewicht.) | | | | | | |

### Beispiel 4

Es wird wie in Beispiel 2 verfahren, jedoch wird eine Lösung aus 1 g Mowiol®4/88 (wasserlöslicher Polyvinylalkohol der Hoechst AG)und 3 g Wasser auf einer Fläche von 476 cm² vorgelegt. Nach der Verarbeitung (vgl. Beispiel 1-3) erhält man einen flexiblen Film mit einem Superabsorberanteil von 189 g /m²; der Anteil an Mowiol®4/88 beträgt 21 g/m². TBT: max./ret. [l/m²]/[l/m²] = 9,4/5,8; AUL (2∗10³Pa) = 5,7 l/m²

### Beispiel 5

Es wird wie in Beispiel 4 verfahren, jedoch wird statt des Mowiol®4/88 Mowiol®5/88 (wasserlöslicher Polyvinylalkohol der Hoechst AG) eingesetzt. Nach der Verarbeitung (vgl. Beispiel 1-3) erhält man einen flexiblen Film mit einem Superabsorberanteil von 144 g/m²; der Anteil an Mowiol®5/88 beträgt 21 g/m².
TBT: max./ret. [l/m²]/[l/m²] = 7,2/4,4; AUL (2∗10³Pa) = 4,4 l/m²

### Beispiel 6

Eine Lösung aus 200 g deionisiertem Wasser, 50 g Glycerin und 10 g Guarkernmehl (Typ 104 der Fa. Roeper) werden zu einer homogenen Lösung verrührt. Die Lösung wird auf eine Fläche von 3000 cm² ausgestrichen und mit 100 g FAVOR®SXM 100 bestreut. Die Fläche wird 4 Stunden bei 75°C getrocknet, anschließend wird das nicht anhaftende SAP mit einem weichen Pinsel entfernt.
Es wird eine absorbierende Fläche mäßiger Flexibilität erhalten mit einem SAP- Anteil von 200 g/m².

### Beispiel 7

Es wird wie in Beispiel 6 verfahren, jedoch wird statt des Guarkernmehls Carboxymethylcellulose (Typ Walocel®40000 der Fa. Wolf Walsrode) verwendet. Außerdem wird 30 Minuten bei 130°C getrocknet. Die dabei erhaltene Fläche ist flexibel und hat einen SAP-Anteil von 180 g/m².

### Beispiel 8

Es wird wie in Beispiel 7 verfahren, jedoch wird statt der Carboxymethylcellulose Acrakonz®BN (lösliches, anvernetztes, anionisches Emulsionspolymerisat auf Basis von Acrylsäurederivaten der Chemischen Fabrik Stockhausen GmbH) verwendet. Entsprechend der Konzentration des Acrakonz®BN wird eine entsprechend höhere Menge (24 g) dieses Produktes eingesetzt. Die dabei erhaltene Fläche ist flexibel und hat einen SAP-Anteil von 240 g/m².

**Tabelle 2:**

| Die Tabelle zeigt die Abhängigkeit der Absorptionsgeschwindigkeit von der Art der eingesetzten Matrixmaterialien. | | | | |
|---|---|---|---|---|
| Bsp. Nr. | SAP | TBT (30 sek.) | TBT (60 sek. ) | TBT (300 sek.) |
| | | max./ret. | max./ret. | max./ret. |
| | [g/m²] | [g/g]/[g/g] | [g/g]/[g/g] | [g/g]/[g/g] |
| 6 | 200 | 6/6 | 15/15 | 21/16 |
| 7 | 180 | 7/7 | 10/10 | 13/13 |
| 8 | 240 | 5/5 | 10/8 | 18/15 |
| (Die TBT Werte beziehen sich auf das Flächengewicht.) | | | | |

### Beispiel 9

Ein Quadratmeter einer Folie aus Polyvinylalkohol (Reel L336; W/O 1483 der Firma Aquafilm Ltd., Dicke 20 µm) wird mit 50 ml einer Lösung aus 50 % Wasser und 50 % Ethylalkohol besprüht und anschließend mit 400 g FAVOR®SXM 100 bestreut. Das Pulver wird leicht an die Oberfläche angedrückt. Anschließend wird 5 Minuten bei 120°C getrocknet.
Der nicht fixierte Teil an SXM 100 wird mit einem Staubsauger entfernt, es verbleiben 113 g/m².
TBT: max./ret. [l/m²]/[l/m²] = 5,7/3,5; AUL (2∗10³Pa) = 3,5 l/m²

### Beispiel 10

Ein Quadratmeter einer Folie aus Polyvinylalkohol (REEL L336; W/O 1483 der Firma Aquafilm Ltd., Dicke 50 µm) wird mit 50 ml einer Lösung aus 50 % Wasser und 50 % Ethylalkohol besprüht und anschließend mit 400 g FAVOR®SXM 100 bestreut. Das Pulver wird leicht an die Oberfläche angedrückt. Anschließend wird 5 Minuten bei 120°C getrocknet.
Der nicht fixierte Teil an SXM 100 wird mit einem Staubsauger entfernt, es verbleiben 179 g/m².
TBT: max./ret. [l/m²]/[l/m²] = 8,9/5,5; AUL (2∗103Pa) = 5,4 l/m²

### Beispiel 11

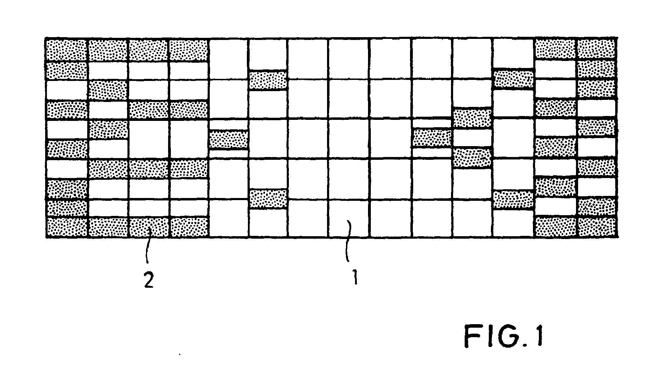

Eine Lösung aus 2g Vinol 205, 2g Glycerin und 6 g Wasser werden auf einer teflonisierten Folie auf einer Fläche von 14 * 44 cm verteilt. Anschließend wird eine Schablone (vgl. Fig. 1) aufgelegt. Die freien Flächen werden mit 9 g Favor SXM 100 bestreut. Die Fläche wird fünf Minuten bei 140°C im Umlufttrockenschrank getrocknet. Anschließend wird sie mit einer Lösung aus 0,25g Vinol 205, 0,25 g Glycerin und 1,5 g Wasser besprüht. Anschließend wird nochmals unter den zuvor genannten Bedingungen getrocknet.
Die so erhaltene Fläche wird von der teflonisierten Folie abgelöst. Sie ist flexibel und zeigt ein Absorptionsvermögen, das dem des eingesetzten Superabsorbers entspricht.

### Beispiel 12

Beispiel 11 wird wiederholt, jedoch wird kein Glycerin verwendet. Die erhaltene Fläche ist hart, spröde und kaum biegbar. Sie zeigt ein Absorptionsvermögen das dem des eingesetzten Superabsorbers entspricht.

### Beispiel 13

Eine Lösung aus 2g Vinol 205, 2g Glycerin und 6 g Wasser werden auf einer teflonisierten Folie auf einer Fläche von 14 ∗ 44 cm verteilt. Anschließend wird eine Schablone (vgl. Fig. 1) aufgelegt. Die freien Flächen werden mit 6 g Favor SXM 100 bestreut. Die Fläche wird fünf Minuten bei 140°C im Umlufttrockenschrank getrocknet.
Anschließend wird sie mit einer Lösung aus 0,25g Vinol 205, 0,25 g Glycerin und 1,5 g Wasser besprüht. Anschließend wird wieder die Schablone aufgelegt. Die freien Flächen werden mit 4.5 g Favor SXM 100 bestreut. Anschließend wird nochmals unter den zuvor genannten Bedingungen getrocknet.
Das Besprühen, Auflegen der Schablone und Aufstreuen des SAP wird noch einmal mit 2,5 g SAP wiederholt.
Letztlich wird noch einmal besprüht und getrocknet. Die so erhaltene Fläche wird von der teflonisierten Folie abgelöst. Sie ist flexibel und zeigt ein Absorptionsvermögen das dem des in allen Raumrichtungen definiert angeordneten Superabsorbers entspricht.

### Beispiel 14

Ein Quadratmeter einer Folie aus Polyvinylalkohol (Reel L336; W/O 1483 der Firma Aquafilm Ltd., Dicke 20 µm) wird mit 50 g FAVOR®SXM 100 bestreut. Das Pulver wird leicht an die Oberfläche angedrückt. Anschließend wird eine zweite Folie aus dem gleichen Material aufgelegt. Die Fläche wird mit einer teflonisierten Folie abdedeckt und mit einem Bügeleisen (Einstellung 180°C) so lange gebügelt, bis die PVA-Folien und der Superabsorber miteinander verschmolzen sind.
Das Absorptionsvermögen der Fläche entspricht der Menge an eingesetztem SAP.

### Beispiel 15

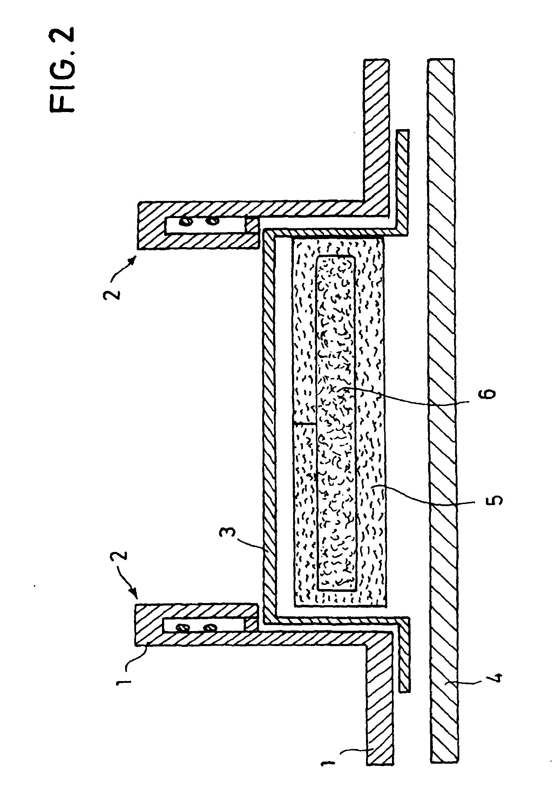

Aus der in Beispiel 13 hergestellten Folie wird gemäß Fig. 2 eine Windel konstruiert.
Die verwendete PE Folie und das Polypropylenabdeckvlies sind Materialien aus einer Windelproduktion.
Die in Beispiel 13 hergestellte Fläche wird als Kern (6) eingesetzt.

### Beispiel 16

10∗15 cm der in Beispiel 2 beschriebenen Fläche werden in eine Verpackungsschale gelegt und mit einem handelsüblichen Küchentuch (Kleenex) abgedeckt. Ein tiefgekühltes Hähnchen (850g) wird auf das Tuch gelegt.
Das gesamte Tauwasser (Testdauer 18 h) wird von der erfindungsgemäßen Fläche aufgesaugt.

### Beispiel 17

Beispiel 12 wird ohne Verwendung einer Schablone wiederholt. Statt des Favors wurd Stockosorb 400 (schwachvernetztes Copolymer auf Basis von Acrylamid) verwendet. Aus dieser Fläche wurden Streifen mit der Größe 1∗7.5 cm ausgeschnitten. Acht der Streifen werden gänzlich in einen Erde enthaltenden zylindrischen Blumentopf (10 cm Höhe, Durchmesser 8,5 cm) gesteckt. Die Erde wurde 5 Tage feucht gehalten. Die Folie hatte sich dannach aufgelöst, das SAP befand sich in einer für z. B. die Pflanzenaufzucht geeigneten Anordnung in der Erde.

### Beispiel 18

Beispiel 1 wird wiederholt, jedoch wird statt des Favors die gleiche Menge der in PCT/EP93/01060 Beispiel 9 genannten superabsorbierenden Depotmittelzubereitung verwendet.
1 cm² der so erhaltenen Fläche werden in einen Teebeutel eingeschweißt. Der Teebeutel wird eine Stunde in 50 ml einer 0,2 % igen Kochsalzlösung eingehängt.
Nach einer Stunde wird die Kochsalzlösung erneuert.
Auch nach dem 5ten Zyklus zeigt die Blaufärbung der Kochsalzlösung eine Freisetztung des Wirkstoffs an.

### Beispiel 19

Eine gemäß Beispiel 10 hergestellte Fläche wird nach dem in Beispiel 14 beschriebenen Verfahren auf ein Gewebe aufgebügelt, wie es für die Ummantelung von Kabeln verwendet wird. Der Verbund der erfindungsgemäßen Fläche mit dem Gewebe ist von hoher mechanischer Festigkeit, das Gewebeband hat seine Flexibilität nicht eingebüßt, die Absorption entspricht dem SAP - Anteil.

Figur 1 zeigt die in Beispiel 11 verwendete Schablone.

Figur 2 zeigt die Windelkonstruktion von Beispiel 15.

## Patentansprüche

1. Flächenförmiges Absorptionsmittel für Wasser oder wäßrige Flüssigkeiten, enthaltend
A) wenigstens ein wasserquellbares, synthetisches und/oder natürliches Polymer und
B) wenigstens ein wasserlösliches, synthetisches und/oder natürliches Polymer,
wobei die Komponente A in der als flächenformig ausgebildeten Matrixkomponenten B definiert ein- oder angebunden ist und wobei das flächenförmige Absorptionsmittel erhältlich ist durch ein Verfahren, bei dem man aus dem wenigstens einen wasserlöslichen synthetischen und/oder natürlichen Polymeren (B) eine flächenförmige Matrix ausbildet und diese mit dem wasserquellbaren synthetischen und/oder natürlichen Polymeren (A) versieht.

2. Flächenförmiges Absorptionsmittel nach Anspruch 1, **dadurch gekennzeichnet, daß** die Komponente A ein Polymer oder Copolymeres auf Basis von (Meth-)acrylsäure, (Meth-)acrylnitril, (Meth-)acrylamid, Vinylacetat, Vinylalkohol, Vinylpyrrolidon, Vinylpyridin, Maleinsäure(anhydrid), Itakonsäure(anhydrid), Fumarsäure, Vinylsulfonsäure, den Amiden, den N-Alkylderivaten, den N,N-Dialkylderivaten und den Estern dieser polymerisierbaren Säuren ist.

3. Flächenförmiges Absorptionsmittel nach Anspruch 1, **dadurch gekennzeichnet, daß** die Komponente A ein schwach vemetztes, natürliches Polymer oder ein Polymer nativen Ursprungs ist wie Guarkemmehl, Carboxymethylcellulose, Xanthan, Alginate, Gummi Arabicum, Chitin, Chitosan, Agar Agar, Hydroxyethylcellulose, Hydroxypropylcellulose, Methylcellulose, Stärke und Stärkederivate oder eine Mischung davon ist.

4. Flächenförmiges Absorptionsmittel nach Anspruch 1, **dadurch gekennzeichnet, daß** Komponente A eine Mischung aus zwei oder mehreren der in den Ansprüchen 2 und 3 genannten Komponenten ist.

5. Flächenförmiges Absorptionsmittel nach Anspruch 1, **dadurch gekennzeichnet, daß** die Komponente B ein wasserlösliches Polymer oder Copolymeres auf Basis von (Meth-)acrylsäure, (Meth-)acrylnitril, (Meth-)acrylamid, Vinylacetat, Vinylalkohol, Vinylpyrrolidon, Vinylpyridin, Maleinsäure(anhydrid), Itakonsäure (-anhydrid), Fumarsäure, Vinylsulfonsäure, den Amiden, den N-Alkylderivaten, den N.N Dialkylderivaten und den Estern dieser polymerisierbaren Säuren ist.

6. Flächenförmiges Absorptionsmittel nach Anspruch 1, **dadurch gekennzeichnet, daß** die Komponente B ein lösliches, natürliches Polymer oder ein Polymer nativen Ursprungs ist wie Guarkernmehl, Carboxymethylcellulose, Xanthan, Alginate, Gummi Arabicum, Chitin, Chitosan, Agar Agar, Hydroxyethylcellulose, Hydroxypropylcellulose, Methylcellulose, Stärke und Stärkederivate oder eine Mischung davon.

7. Flächenformiges Absorptionsmittel nach den Ansprüchen 1,5,6, **dadurch gekennzeichnet, daß** Komponente B eine Mischung aus den Komponenten nach den Ansprüchen 5 und 6 ist.

8. Flächenförmiges Absorptionsmittel nach einem der vorhergehenden Ansprüche, **dadurch gekennzeichnet, daß** das Verhältnis von B : A = 1 : 1000 bis 100 : 1 liegt,. bevorzugt sind Verhältnisse von B : A = 1 : 100 bis 10 : 1, und besonders bevorzugt B : A = 1 : 25 bis 2 : 1.

9. Flächenförmiges Absorptionsmittel nach einem der Ansprüche 1 - 8, **dadurch gekennzeichnet, daß** die Komponenten A und B teilweise chemisch miteinander reagiert haben.

10. Flächenförmiges Absorptionsmittel nach einem der Ansprüche 1 - 9, **dadurch gekennzeichnet, daß** die Komponenten A und B rein physikalisch miteinander verknüpft sind.

11. Flächenförmiges Absorptionsmittel nach einem der vorhergehenden Ansprüche in Form eines Films, eines Blattes, einer Folie oder eines Rollgutes, eines Laminats oder eines anderen Schichtproduktes .

12. Verfahren zur Herstellung eines flächenförmigen Absorptionsmittels nach einem der Ansprüche 1 bis 11, **dadurch gekennzeichnet, daß** man aus wenigstens einem wasserlöslichen synthetischen und/oder natürlichen Polymeren (B) eine flächenförmige Matrix ausbildet und diese mit einem wasserquellbaren synthetischen und/oder natürlichen Polymeren (A) versieht.

13. Verfahren nach Anspruch 12, **dadurch gekennzeichnet, daß** man
a) eine Lösung der Komponente B in Wasser oder einer wäßrigen Lösung herstellt,
b) diese Lösung auf eine Fläche aufbringt,
c) diese Fläche mit Komponente A bestreut und
d) die erhaltene Flächenstruktur trocknet.

14. Verfahren nach Anspruch 13, **dadurch gekennzeichnet, daß** man die Schritte b und c mehrmals, gegebenenfalls mit zwischengeschalteten Trocknungen, wiederholt.

15. Verfahren nach Anspruch 13 - 14, **dadurch gekennzeichnet, daß** man das flächenförmige Absorptionsmittel abschließend gemäß Anspruch 13 Schritte b und d behandelt.

16. Verfahren nach Anspruch 13 - 15, **dadurch gekennzeichnet, daß** man die Lösung der Komponente B durch Streichen, Rakeln, Sprühen, Gießen, Flatschen oder Beträufeln aufbringt.

17. Verfahren nach Anspruch 12, **dadurch gekennzeichnet, daß** man
a) eine Folie aus Komponente B mit einem Stoff aus Komponenten A in Kontakt bringt,
b) ein für Komponente B geeignetes Lösungsmittel zusetzt, daß sich die vorgelegte Folie anlöst, nicht aber auflöst und
c) das so entstandene Produkt trocknet.

18. Verfahren nach Anspruch 17, **dadurch gekennzeichnet, daß** man zunächst ein für Komponente B geeignetes Lösungsmittel zugesetzt und dann mit der Komponente A bestreut.

19. Verfahren nach Anspruch 17 oder 18, **dadurch gekennzeichnet, daß** man die Schritte a, b mehrmals wiederholt.

20. Verfahren nach Anspruch 17 bis 19, **dadurch gekennzeichnet, daß** man flächenförmige Absorptionsmittel vor oder nach dem letzten Trocknen mit einer Folie aus Komponente B abgedeckt.

21. Verfahren nach Anspruch 17 bis 20, **dadurch gekennzeichnet, daß** man das Lösungsmittel für Komponente B durch Streichen, Rakeln, Sprühen, Gießen, Flatschen oder Beträufeln aufbringt.

22. Verfahren nach Anspruch 13, **dadurch gekennzeichnet, daß** man
a) ein für Komponente B geeignetes Lösungsmittel mit Komponenten A mischt,
b) eine Folie aus Komponente B mit dieser Mischung kontaktiert und
c) das so erhaltene Produkt trocknet.

23. Verfahren nach Anspruch 22, **dadurch gekennzeichnet, daß** man die Schritte a, b mehrmals wiederholt.

24. Verfahren nach Anspruch 22 oder 23, **dadurch gekennzeichnet, daß** man das aus Stufe c erhaltene Produkt abschließend mit einer Folie aus Komponente B abdeckt.

25. Verfahren nach Anspruch 22 bis 24, **dadurch gekennzeichnet, daß** man das Lösungsmittel für Komponente B auf Komponente A durch Sprühen, Gießen, Flatschen oder Beträufeln aufbringt.

26. Verfahren nach Anspruch 13, **dadurch gekennzeichnet, daß** man
a) eine Folie aus Komponente B mit einem Polymeren aus Komponente A bestreut und
b) das Polymere gemäß Anspruch 2 bis 4 thermisch und/oder unter Druck in die Folie fixiert.

27. Verfahren nach Anspruch 26, **dadurch gekennzeichnet, daß** man das in Stufe b) erhaltene Produkt vor einer Wärmebehandlung mit einer weiteren Folie aus Komponente B abdeckt.

28. Verfahren nach Anspruch 25, **dadurch gekennzeichnet, daß** man das in Stufe b) erhaltene Produkt nach der Wärmebehandlung mit einer Folie abdeckt.

29. Verfahren nach Anspruch 26 oder 27, **dadurch gekennzeichnet, daß** man die Verfahrensschritte beliebig oft wiederholt.

30. Verfahren zur Herstellung eines flächenförmigen Absorptionsmittels nach Anspruch 1 bis 11, **dadurch gekennzeichnet, daß** man Komponente A nach dem Extrudieren von Komponente B in diese einbringt.

31. Verfahren nach Anspruch 13 bis 30, **dadurch gekennzeichnet, daß** man zum Aufstreuen der Komponente A eine Schablone verwendet, welche eine definierte Anordnung der Komponente A in der Komponente B ermöglicht.

32. Verwendung der Wasser und wäßrige Flüssigkeiten absorbierenden Fläche nach den Ansprüchen 1 bis 11 in Hygieneartikeln im sanitären und medizinischen Bereich zur Aufnahme von Wasser oder Körperflüssigkeiten.

33. Verwendung der Wasser und wäßrige Flüssigkeiten absorbierenden Fläche nach den Ansprüchen 1 bis 11 direkt oder als Komponente in natürlichen und/oder künstlichen Böden zur Pflanzenzucht oder zum Transport und zur Lagerung von Pflanzen oder Pflanzenteilen.

34. Verwendung der Wasser und wäßrige Flüssigkeiten absorbierenden Fläche nach den Ansprüchen 1 bis 11 als wasserblockierendes Isolationsmaterial fiir Rohre und Leitungen, insbesondere für elektrische und lichtleitende Kabel.

35. Verwendung der Wasser und wäßrige Flüssigkeiten absorbierenden Fläche nach den Ansprüchen 1 bis 11 als wasserblockierendes Isolationsmaterial fiir Baukonstruktionen, insbesondere für Außenmauern.

36. Verwendung der Wasser und wäßrige Flüssigkeiten absorbierenden Fläche nach den Ansprüchen 1 bis 11 direkt oder als flüssigkeitsaufnehmende und/oder flüssigkeitsspeichernde Komponente in Verpackungsmaterialien.

37. Verwendung der Wasser und wäßrige Flüssigkeiten absorbierenden Fläche nach den Ansprüchen 1 bis 11 als Teil in Bekleidungsstücken.

38. Verwendung der Wasser und wäßrige Flüssigkeiten absorbierenden Fläche nach den Ansprüchen 1 bis 11 als Speicher zur kontrollierten Freisetzung eines Wirkstoffs.

39. Chemisch-technische Produkte, enthaltend eine Zusammensetzung nach den Ansprüchen 1 bis 11 oder hergestellt gemäß den Ansprüchen 12 bis 14.

## Claims

1. Sheet-like absorbent for water or aqueous fluids, containing
A) at least one water-swellable, synthetic and/or natural polymer and
B) at least one water-soluble, synthetic and/or natural polymer,
wherein component A is integrated or attached in a defined manner in the matrix component B designed so as to be sheet-like and wherein the sheet-like absorbent is obtainable by a process in which a sheet-like matrix is formed from the at least one water-soluble synthetic and/or natural polymer (B) and this is provided with the water-swellable synthetic and/or natural polymer (A).

2. Sheet-like absorbent according to claim 1, **characterised in that** component A is a polymer or copolymer based on (meth)acrylic acid, (meth)acrylonitrile, (meth)acrylamide, vinyl acetate, vinyl alcohol, vinyl pyrrolidone, vinyl pyridine, maleic acid(anhydride), itaconic acid(anhydride), fumaric acid, vinyl sulphonic acid, the amides, the N-alkyl derivatives, the N,N-dialkyl derivatives and the esters of these polymerisable acids.

3. Sheet-like absorbent according to claim 1, **characterised in that** component A is a weakly crosslinked, natural polymer or a polymer of natural origin, such as guar gum, carboxymethylcellulose, xanthan gum, alginates, gum arabic, chitin, chitosan, agar agar, hydroxyethylcellulose, hydroxypropylcellulose, methylcellulose, starch and starch derivatives or a mixture thereof.

4. Sheet-like absorbent according to claim 1, **characterised in that** component A is a mixture of two or more of the components mentioned in claims 2 and 3.

5. Sheet-like absorbent according to claim 1, **characterised in that** component B is a water-soluble polymer or copolymer based on (meth)acrylic acid, (meth)acrylonitrile, (meth)acrylamide, vinyl acetate, vinyl alcohol, vinyl pyrrolidone, vinyl pyridine, maleic acid(anhydride), itaconic acid(anhydride), fumaric acid, vinyl sulphonic acid, the amides, the N-alkyl derivatives, the N,N-dialkyl derivatives and the esters of these polymerisable acids.

6. Sheet-like absorbent according to claim 1, **characterised in that** component B is a soluble, natural polymer or a polymer of natural origin, such as guar gum, carboxymethylcellulose, xanthan gum, alginates, gum arabic, chitin, chitosan, agar agar, hydroxyethylcellulose, hydroxypropylcellulose, methylcellulose, starch and starch derivatives or a mixture thereof.

7. Sheet-like absorbent according to claims 1, 5, 6, **characterised in that** component B is a mixture of the components according to claims 5 and 6.

8. Sheet-like absorbent according to any of the preceding claims, **characterised in that** the ratio of B : A = 1 : 1,000 to 100 : 1, ratios of B : A = 1 : 100 to 10 : 1 being preferred and B : A = 1 : 25 to 2 : 1 being particularly preferred.

9. Sheet-like absorbent according to any of claims 1 to 8, **characterised in that** components A and B have partially chemically reacted with one another.

10. Sheet-like absorbent according to any of claims 1 to 9, **characterised in that** components A and B are coupled purely physically to one another.

11. Sheet-like absorbent according to any of the preceding claims in the form of a film, a leaf, a foil or a rolled product, a laminate or another layered product.

12. Method for producing a sheet-like absorbent according to any of claims 1 to 11, **characterised in that** a sheet-like matrix is formed from at least one water-soluble synthetic and/or natural polymer (B) and is provided with a water-swellable, synthetic and/or natural polymer (A).

13. Method according to claim 12, **characterised in that**
a) a solution of component B is produced in water or an aqueous solution,
b) this solution is applied to a sheet,
c) this sheet is sprinkled with component A,
d) the sheet structure obtained is dried.

14. Method according to claim 13, **characterised in that** steps b and c are repeated several times optionally with interposed dryings.

15. Method according to claims 13 to 14, **characterised in that** the sheet-like absorbent is finally treated in accordance with claim 13, steps b and d.

16. Method according to claims 13 to 15, **characterised in that** the solution of component B is applied by coating, doctoring, spraying, pouring, slop padding or dripping.

17. Method according to claim 12, **characterised in that**
a) a film made of component B is brought into contact with a material made of contact A,
b) a solvent suitable for component B is added so the presented film is attacked but does not dissolve and
c) the resulting product is dried.

18. Method according to claim 17, **characterised in that** a solvent suitable for component B is initially added and then component A is sprinkled over.

19. Method according to claim 17 or 18, **characterised in that** steps a, b are repeated several times.

20. Method according to claims 17 to 19, **characterised in that** sheet-like absorbents are covered before or after the final drying with the film made of component B.

21. Method according to claims 17 to 20, **characterised in that** the solvent for component B is applied by coating, doctoring, spraying, pouring, slop padding or dripping.

22. Method according to claim 13, **characterised in that**
a) a solvent suitable for component B is mixed with component A,
b) a film made of component B is brought into contact with this mixture and
c) the resulting product is dried.

23. Method according to claim 22, **characterised in that** steps a, b are repeated several times.

24. Method according to claims 22 or 23, **characterised in that** the product obtained from stage c is finally covered with a film made of component B.

25. Method according to claims 22 to 24, **characterised in that** the solvent for component B is applied to component A by spraying, pouring, slop padding or dripping.

26. Method according to claim 13, **characterised in that**
a) a film made of component B is sprinkled with a polymer made of component A and
b) the polymer according to claims 2 to 4 is fixed thermally and/or under pressure into the film.

27. Method according to claim 26, **characterised in that** the product obtained in stage b) is covered by a further film made of component B before a heat treatment.

28. Method according to claim 25, **characterised in that** the product obtained in stage b) is covered with a film after the heat treatment.

29. Method according to claim 26 or 27, **characterised in that** the method steps are repeated as often as desired.

30. Method for producing a sheet-like absorbent according to claims 1 to 11, **characterised in that** component A is introduced into component B after extrusion of the latter.

31. Method according to claims 13 to 30, **characterised in that** a template is used for spreading component A, allowing defined arrangement of component A in component B.

32. Use of the water- and aqueous fluid-absorbing sheet according to claims 1 to 11 in hygienic articles in the hygiene and medical sector for absorbing water or bodily fluids.

33. Use of the water- and aqueous fluid-absorbing sheet according to claims 1 to 11 directly or as a component in natural and/or synthetic soils for the cultivation of plants or for transporting and storing plants or plant parts.

34. Use of the water- and aqueous fluid-absorbing sheet according to claims 1 to 11 as water-blocking insulating material for pipes and lines, in particular for electrical and light-guiding cables.

35. Use of the water- and aqueous fluid-absorbing sheet according to claims 1 to 11 as water-blocking insulating material for building constructions, in particular for external walls.

36. Use of the water- and aqueous fluid-absorbing sheet according to claims 1 to 11, directly or as fluid-absorbing and/or fluid-storing components in packaging materials.

37. Use of the water- and aqueous fluid-absorbing sheet according to claims 1 to 11 as a component in clothing.

38. Use of the water- and aqueous fluid-absorbing sheet according to claims 1 to 11 as storage medium for controlled release of an active ingredient.

39. Chemical-technical products containing a composition according to claims 1 to 11 or produced in accordance with claims 12 to 14.

## Revendications

1. Agent d'absorption en forme de feuilles pour de l'eau ou des liquides aqueux, contenant
A) au moins un polymère synthétique et/ou naturel gonflant dans l'eau,
et
B) au moins un polymère synthétique et/ou naturel soluble dans l'eau,
dans lequel le composant A est incorporé ou attaché de manière définie dans le composant matriciel B réalisé de manière en forme de feuilles, et dans lequel l'agent d'absorption en forme de feuilles peut être obtenu par un procédé, dans lequel on forme de l'au moins un polymère synthétique et/ou naturel soluble dans l'eau (B), une matrice et on dote celle-ci avec le polymère synthétique et/ou naturel gonflant dans l'eau (A).

2. Agent d'absorption en forme de feuilles selon la revendication 1, **caractérisé en ce que** le composant A est un polymère ou un copolymère à base d'acide (meth)acrylique, de (méth)acrylonitrile, de (méth)acrylamide, d'acétate de vinyle, d'alcool vinylique, vinylpyrrolidone, de vinylpyridine, d'acide(anhydride)maléique, d'acide(anhydride) itaconique, d'acice fumarique, d'acide vinylsulfonique, ainsi que des amides, des dérivés N-alkyle, des dérivés N,N-dialkyle et des esters de ces acides polymérisables.

3. Agent d'absorption en forme de feuilles selon la revendication 1, **caractérisé en ce que** le composant est un polymère naturel faiblement réticulé ou un polymère d'origine naturelle tel que la farine guar, la carboxyméthylcellulose, le xanthane, les alginates, la gomme arabique, la chitine, le chitosan, l'agar, l'hydroxyéthylcellulose, l'hydroxypropylcellulose, la méthylcellulose, l'amidon et les dérivés de l'amidon ou un mélange de ceux-ci.

4. Agent d'absorption en forme de feuilles selon la revendication 1, **caractérisé en ce que** le composant A est un mélange de deux ou plusieurs composants mentionnés dans les revendications 2 et 3.

5. Agent d'absorption en forme de feuilles selon la revendication 1, **caractérisé en ce que** le composant B est un polymère ou un copolymère soluble dans l'eau à base d'acide (méth)acrylique, de (méth)acrylonitrile, de (méth)acrylamide, d'acétate de vinyle, d'alcool vinylique, de vinylpyrrolidone, de vinylpyridine, d'acide(anhydride)maléique, d'acide(anhydride) itaconique, d'acide fumarique, d'acide vinylsulfonique, des amides, des dérivés N,N-dialyle et des esters de ces acides polymérisables.

6. Agent d'absorption en forme de feuilles selon la revendication 1, **caractérisé en ce que** le composant B est un polymère naturel soluble dans l'eau ou un polymère d'origine naturelle tel que la farine de guar, la carboxyméthylcellulose, le xanthane, les alginates, la gomme arabique, la chitine, le chitosan, l'agar agar, l'hydroxyéthylcellulose, l'hydroxypropylcellulose, la méthylcellulose, l'amidon et les dérivés de l'amidon ou un mélange de ceux-ci.

7. Agent d'absorption en forme de feuilles selon selon les revendications 1, 5, 6, **caractérisé en ce que** le composant B est un mélange des composants selon les revendications 5 et 6.

8. Agent d'absorption en forme de feuilles selon l'une quelconque des revendications précédentes, **caractérisé en ce que** le rapport B/A est de 1/1000 à 100/1, les rapports de B/A de 1/100 à 10/1 sont préférés et les rapports B/A de 1/25 à 2/1 sont particulièrement préférés.

9. Agent d'absorption en forme de feuilles selon l'une quelconque des revendications 1 à 8, **caractérisé en ce que** les composants A et B ont réagi partiellement de manière chimique l'un avec l'autre.

10. Agent d'absorption en forme de feuilles selon l'une quelconque des revendications 1 à 9, **caractérisé en ce que** les composants A et B sont liés entre eux de manière purement physique.

11. Agent d'absorption en forme de feuilles selon l'une quelconque des revendications précédentes sous forme d'un film, d'une feuille, d'une feuille mince ou de matière en rouleau, de stratifié ou d'autre produit en couches.

12. Procédé de fabrication d'un agent d'absorption en forme de feuilles selon l'une quelconque des revendications 1 à 11, **caractérisé en ce qu'**on forme une matrice en forme de feuilles à partir d'au moins un polymère synthétique et/ou naturel soluble dans l'eau (B) et on applique sur cette matrice un polymère synthétique et/ou naturel gonflant dans l'eau (A).

13. Procédé selon la revendication 12, **caractérisé en ce que**
a) on prépare une solution du composant B dans l'eau ou dans une solution aqueuse,
b) on applique cette solution sur une surface,
c) on répand sur cette surface le composant A, et
d) on sèche la structure surfacique obtenue.

14. Procédé selon la revendication 13, **caractérisé en ce qu'**on répète plusieurs fois les phases b et c, le cas échéant avec des séchages intérmédiaires.

15. Procédé selon les revendications 13 à 14, **caractérisé en ce qu'**on traite finalement l'agent d'absorption en forme de feuilles selon la revendication 13, phases b et d.

16. Procédé selon les revendications 13 à 15, **caractérisé en ce qu'**on applique la solution du composant b par enduction, raclage, pulvérisation, coulée, aspersion ou versement goutte à goutte.

17. Procédé selon la revendication 12, **caractérisé en ce que**
a) on met en contact une feuille du composant B avec une matière du composant A,
b) on ajoute un solvant approprié pour le composant B, de telle sorte que la feuille précédemment appliquée se soude, mais ne se dissout pas, et
c) on sèche le produit ainsi obtenu.

18. Procédé selon la revendication 17, **caractérisé en ce qu'**on ajoute d'abord un solvant approprié pour le composant B et on répand ensuite sur celui-ci le composant A.

19. Procédé selon la revendication 17 ou 18, **caractérisé en ce qu'**on répète plusieurs fois les phases a, b.

20. Procédé selon les revendications 17 à 19, **caractérisé en ce qu'**on recouvre l'agent d'absorption en forme de feuilles avant ou après le dernier séchage avec une feuille du composant B.

21. Procédé selon la revendication 17 à 20, **caractérisé en ce qu'**on applique le solvant pour le composant B par enduction, raclage, pulvérisation, coulée, aspersion ou versement goutte à goutte.

22. Procédé selon la revendication 13, **caractérisé en ce que**
a) on mélange avec le composant A un solvant approprié pour le composant B,
b) on met en contact une feuille du composant B avec ce mélange et
c) on sèche le produit ainsi obtenu.

23. Procédé selon la revendication 22, **caractérisé en ce qu'**on répète plusieurs fois les phases a, b.

24. Procédé selon les revendications 22 ou 23, **caractérisé en ce qu'**on recouvre finalement avec une feuille du composant B le produit obtenu dans la phase c.

25. Procédé selon les revendications 22 à 24, **caractérisé en ce qu'**on applique le solvant pour le composant B sur le composant A par pulvérisation, coulée, aspersion ou versement goutte par goutte.

26. Procédé selon la revendication 13, **caractérisé en ce que**
a) on répand sur une feuille de composé B un polymère du composant A, et
b) on fixe dans la feuille le polymère selon les revendications 1 à 4 thermiquement et/ou sous pression.

27. Procédé selon la revendication 26, **caractérisé en ce qu'**on recouvre le produit obtenu dans la phase b) avant un traitement thermique avec une autre feuille du composant B.

28. Procédé selon la revendication 26, **caractérisé en ce qu'**on recouvre avec une feuille, après le traitement thermique, le produit obtenu dans la phase b).

29. Procédé selon les revendications 26 ou 27, **caractérisé en ce qu'**on répète aussi souvent que l'on veut les phases du procédé.

30. Procédé de fabrication d'un agent d'absorption en forme de feuilles selon les revendications 1 à 11, **caractérisé en ce qu'**on introduit le composant A dans le composant B après l'extrusion dans ce dernier.

31. Procédé selon les revendications 13 à 30, **caractérisé en ce qu'**on utilise pour l'application du composant A, un pochoir qui permet und disposition définie du composant A dans le composant B.

32. Utilisation de la structure absorbant l'eau et les liquides aqueux selon les revendications 1 à 11 dans des articles d'hygiène, dans les domaines sanitaire et médical, pour l'absorption d'eau ou de liquides corporels.

33. Utilisation de la structure absorbant l'eau et les liquides aqueux selon les revendications 1 à 11 directement ou en tant que composant dans les sols naturels et/ou artificiels pour la culture de plantes ou pour le transport et pour le stockage de plantes ou de parties de plantes.

34. Utilisation de la structure absorbant l'eau et les liquides aqueux selon les revendications 1 à 11 en tant que matériau isolant bloquant l'eau pour les tubes et conduites, notamment pour les câbles électriques et d'éclairage.

35. Utilisation de la structure absorbant l'eau et les liquides aqueux selon les revendications 1 à 11 en tant que matériau isolant bloquant l'eau pour les constructions dans le bâtiment, notamment pour les murs extérieurs.

36. Utilisation de la structure absorbant l'eau et les liquides aqueux selon les revendications 1 à 11 directement ou en tant que composant absorbant les liquides et/ou retenant les liquides dans les matériaux d'emballage.

37. Utilisation de la structure absorbant l'eau et les liquides aqueux selon les revendications 1 à 11 en tant que pièces de vêtements.

38. Utilisation de la structure absorbant l'eau et les liquides aqueux selon les revendications 1 à 11 en tant que réservoir pour la libération contrôlée d'un principe actif.

39. Produits chimico-techniques contenant une composition selon les revendications 1 à 11 ou fabriqués selon les revendications 12 à 14.
